# EUROPEAN PATENT APPLICATION

(11) **EP 4 755 296 A1**
(43) Date of publication of application: **10.06.2026**
(21) Application number: 24217684.0
(22) Date of filing: 05.12.2024
(51) Int. Cl.: A61B 5/01, A61B 5/145, A61B 5/00

(54) **SECURING DEVICE**

(71) Applicant: Syai UK Ltd, Cambridge CB2 8DL (GB)
(72) Inventor: WANG, Zhihua, Cambridge, CB2 8DL (GB)
(74) Representative: Nederlandsch Octrooibureau

(57) **Abstract**

The present application relates to the technical field of medical devices and discloses a securing device for improving the protection of a monitoring device. The securing device comprises an application part and a support part, and the support part is connected to the application part. The application part is used to affix to the skin surface of the user, and the application part is configured with an avoidance hole for avoiding the monitoring device. The support part comprises a support structure, an accommodating space is arranged in the interior of the support structure to receive the monitoring device, an opening is configured on a side of the support structure that is close to the application part, and the opening is directly opposite to the avoidance hole. The support structure comprises a plurality of reinforcing ribs, the plurality of reinforcing ribs are spaced in a circumferential direction around the first avoidance hole. Along the radial direction of the avoidance hole, a first end of each of the reinforcing ribs is connected to the application part, a second end of each of the reinforcing ribs extends towards a center of the avoidance hole, and the second ends of the reinforcing ribs are interconnected so that the plurality of reinforcing ribs coordinately form the accommodating space.

## Description

### TECHNICAL FIELD

The present application relates to the technical field of medical devices, and particularly to a securing device.

### BACKGROUND

With the improvement of living standards, people pay more and more attention to their health. For the purpose of health monitoring, users need to wear some monitoring devices to monitor various physical changes. During the monitoring process, the monitoring devices need to be secured by means of securing devices, which secure the monitoring devices onto the skin surface of the users. However, some of the commercially available securing devices cannot well protect the monitoring devices, especially when the users are in motion or subjected to external forces, the products are easily shifted or damaged under the action of external forces. As a result, the monitoring results of the monitoring devices are affected, leading to a poor user experience.

### SUMMARY

The present application provides a securing device that may improve the protection effect of the monitoring device and guarantee the user experience.

The present application provides a securing device comprising an application part and a support part, and the support part is connected to the application part;
the application part is used to affix to the skin surface of the user, and the application part is configured with an avoidance hole for avoiding a monitoring device;
the support part comprises a support structure, an accommodating space is arranged in the interior of the support structure to receive the monitoring device, an opening is configured on a side of the support structure that is close to the application part, and the opening is directly opposite to the avoidance hole; and
the support structure comprises a plurality of reinforcing ribs, the plurality of reinforcing ribs are spaced in a circumferential direction around the first avoidance hole; and along the radial direction of the avoidance hole, a first end of each of the reinforcing ribs is connected to the application part, a second end of each of the reinforcing ribs extends towards a center of the avoidance hole, and the second ends of the reinforcing ribs are interconnected so that the plurality of reinforcing ribs coordinately form the accommodating space.

According to the securing device provided by the present application, the application part is used to affix to the skin surface of the user, and the support part is used to restrict and protect the monitoring device. The support structure has the accommodating space, and the accommodating space is formed by the plurality of reinforcing ribs that are interconnected. Since the second ends of the reinforcing ribs are interconnected, when the monitoring device is in the accommodating space, the accommodating space is cooperated with the opening to restrict the monitoring device, thus preventing the monitoring device from falling out of the support structure. In addition, since the second ends of the reinforcing ribs extend to a center of the avoidance hole, when the second ends of the reinforcing ribs are interconnected, a position limiting structure is formed on the top of the monitoring device, and the position limiting structure also prevents an external force from being directly applied to the surface of the monitoring device, thus avoiding scratching or rubbing the monitoring device. Therefore, the securing device of the present application may not only positionally restrict and protect the monitoring device, but also prevent the monitoring device from being scratched and rubbed, thereby improving the protection effect of the monitoring device and guaranteeing the user experience.

In some possible embodiments, when the monitoring device is located in the accommodating space, at least a portion of each of the reinforcing ribs is in surface contact with the monitoring device.

In some possible embodiments, an orthographic projection of the opening on a projection plane coincides with an orthographic projection of the monitoring device on the projection plane, the projection plane is a plane perpendicular to an axis of the avoidance hole.

In some possible embodiments, the second ends of the reinforcing ribs are in surface contact with the top of the monitoring device.

In some possible embodiments, the support part further comprises a connecting structure, and the support structure is connected to the application part by means of the connecting structure; and the connecting structure is configured with the opening, and the first ends of the reinforcing ribs are connected to an inner wall of the opening.

In some possible embodiments, the connecting structure and the support structure are integrated in one-piece construction.

In some possible embodiments, the reinforcing ribs are integrated in one-piece construction.

In some possible embodiments, a portion of each of the reinforcing ribs that is close to the first end is configured with a hollow.

In some possible embodiments, the application part comprises a substrate layer, a bonding layer, and a release layer stacked in sequence, the support part is connected to a side of the substrate layer which faces away from the bonding layer, and the release layer covers the bonding layer.

In some possible embodiments, the application part further comprises a bonding clearance layer, the bonding clearance layer is disposed on a side of the bonding layer which faces away from the substrate layer; and the bonding layer and the bonding clearance layer are in ring-shaped structures, the bonding layer is coaxially disposed with respect to the bonding clearance layer, and along the radial direction of the bonding layer, an outer ring edge of the bonding clearance layer is located between an outer ring edge of the bonding layer and an inner ring edge of the bonding layer.

### BRIEF DESCRIPTION OF DRAWINGS

FIG. 1 is a structural diagram of a securing device receiving a monitoring device in an embodiment of the present application;
FIG. 2 is an overall structural diagram of a securing device in an embodiment of the present application;
FIG. 3 is a structural diagram of a support part in an embodiment of the present application;
FIG. 4 is a bottom view of the support part in FIG. 3;
FIG. 5 is an exploded structural diagram of the securing device in an embodiment of the present application;
FIG. 6 is a process state diagram of covering the monitoring device with the securing device in an embodiment of the present application; and
FIG. 7 is a state diagram of covering the monitoring device with the securing device in an embodiment of the present application.

In the drawings:
10-monitoring device; 100-application part; 101-avoidance hole; 110-substrate layer; 120-bonding layer; 130-bonding clearance layer; 140-release layer; 141-first release part; 142-second release part; 143-peel-off tab; 200-support part; 210-connecting structure; 211-opening; 220-support structure; 221-reinforcing rib; 2211-first reinforcing portion; 2212-second reinforcing portion; 222-position limiting surface; 223-hollow.

### DETAILED DESCRIPTION OF THE EMBODIMENTS

The technical solutions in the embodiments of the present application will be described clearly and completely in connection with the drawings in the embodiments of the present application in the following paragraphs. Apparently, the embodiments described are only some, not all of the embodiments of the present application. All other embodiments obtained by those of ordinary skill in the art without creative work based on the embodiments of the present application are within the scope of protection of the present application.

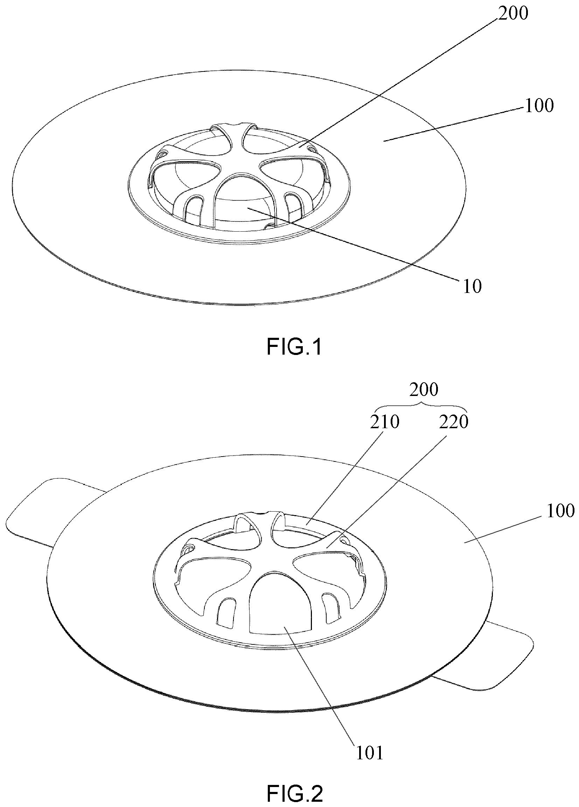

Referring to FIG. 1, a securing device in the embodiment of the present application may comprise an application part 100 and a support part 200, and the support part 200 is connected to the application part 100. Wherein, the application part 100 is used to affix to the skin surface of the user, and the support part 200 is used to support a monitoring device 10 worn on the skin surface of the user, and under the joint action of the application part 100 and the support part 200, a protective effect on the monitoring device 10 is achieved.

Specifically, referring together to FIGs. 1 and 2, the application part 100 has opposing first and second sides, the first side contacts the skin surface of the user, and the second side is used to connect the support part 200. The application part 100 may be configured with an avoidance hole 101. When the securing device in this embodiment is affixed to the skin surface of the user, the monitoring device may pass through the avoidance hole 101, at which point the application part 100 is in close contact with the skin of the user, so that the securing device is securely affixed to the skin surface of the user, and the application part 100 does not cover the monitoring device 10, thus avoiding worsening the breathability of the monitoring device 10. Referring together to FIGs. 2 to 4, the support part 200 comprises a connecting structure 210 and a support structure 220, wherein the connecting structure 210 may be directly fixed onto the second side of the application part 100, and the support structure 220 is connected to a side of the connecting structure 210 which faces away from the application part 100. The connecting structure 210 may be ring-shaped with an opening 211 configured in a mid-portion thereof, the opening 211 is directly opposite to the avoidance hole 101. Exemplarily, the opening 211 and the avoidance hole 101 may have the same dimensions, at which point an inner wall of the opening 211 is aligned with an inner wall of the avoidance hole 101, so that the monitoring device 10 may extend into the support structure 220 after passing through the opening 211.

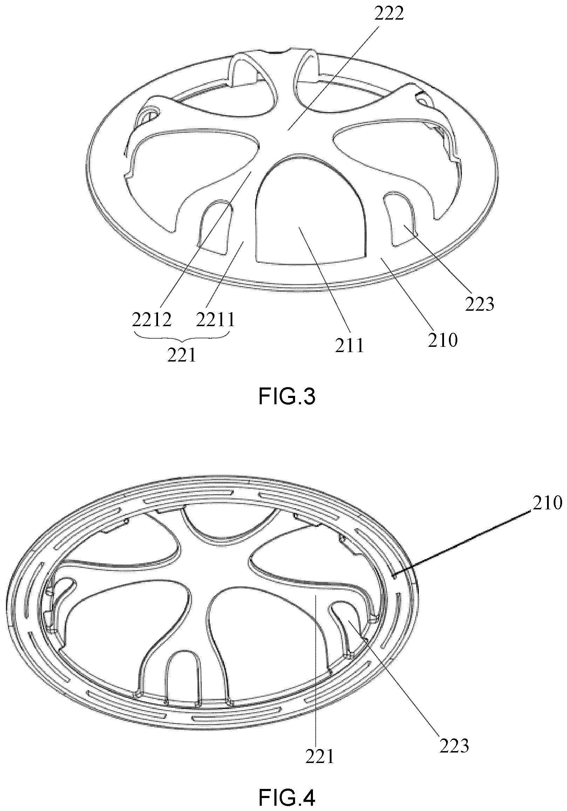

As shown in FIG. 3, the support structure 220 may comprise a plurality of reinforcing ribs 221, and the plurality of reinforcing ribs 221 are spaced in the circumferential direction around the opening 211. Along the radial direction of the opening 211, a first end of each of the reinforcing ribs 221 is connected to the inner wall of the opening 211, a second end extends towards a center of the opening 211, and the second ends of the reinforcing ribs 221 are interconnected so that the reinforcing ribs 221 coordinately form an accommodating space for receiving the monitoring device 10.

It is understood that when the monitoring device 10 extends into the accommodating space through the opening 211, since the second ends of the reinforcing ribs 221 are interconnected, a position limiting structure is formed on the top surface of the monitoring device 10 so that the monitoring device 10 is prevented from falling from a side of the support structure 220 which faces away from the opening 211, thus effectively restricting the monitoring device 10. In addition, since the second ends of the reinforcing ribs 221 extend towards the center of the opening 211, an interconnected part of the second ends of the reinforcing ribs 221 partially covers the top surface of the monitoring device 10. When there is an external force applied to a side of the support structure 220 which faces away from the application part 100, the reinforcing ribs 221 may also play a role in protecting the monitoring device 10 by preventing the external force from being directly applied to the monitoring device 10, thereby preventing the monitoring device 10 from being scratched or damaged.

Continuously referring to FIG. 3, the reinforcing ribs 221 may have a substantial L-shape. Specifically, each of the reinforcing ribs 221 may comprise a first reinforcing portion 2211 and a second reinforcing portion 2212 that are interconnected. The first reinforcing portion 2211 is connected to the inner wall of the opening 211, and one end of each the second reinforcing portion 2212 is connected to the first reinforcing portion 2211, and the other end of each the second reinforcing portion 2212 is interconnected. The first reinforcing portion 2211 may extend along a first preset direction, which may be parallel to an axis of the opening 211. Alternatively, the first preset direction forms a small angle with respect to the axis of the opening 211 to allow for a certain spacing between the second reinforcing portion 2212 and the connecting structure 210 in the axial direction of the opening 211.

The second reinforcing portion 2212 may extend along a second preset direction, which may be perpendicular to the axis of the opening 211. Alternatively, the second preset direction forms a small angle with respect to the direction which is perpendicular to the axis of the opening 211. In this way, when the second reinforcing portions 2212 are interconnected, a relatively flat position limiting surface 222 may be formed to facilitate restricting the monitoring device 10.

In some embodiments, a projection plane is preset, which may be regarded as a plane perpendicular to an axis of the avoidance hole 101. When the monitoring device 10 extends into the accommodating space through the opening 211, an orthographic projection of the monitoring device 10 on the projection plane may coincide with an orthographic projection of the opening 211 on the projection plane, so that the opening 211 may contact with an outer surface of the monitoring device 10 along the circumferential direction of the monitoring device 10, thus achieving the effect of restricting the monitoring device 10 in the radial direction.

Further, at least a portion of the reinforcing rib 221 may also be in surface contact with the monitoring device 10, so as to achieve the effect of restricting the monitoring device 10 in the radial or axial direction. Exemplarily, in conjunction with FIGs. 1 and 3, the first reinforcing portion 2211 may extend along a side profile of the monitoring device 10, so that the first reinforcing portion 2211 may fit to the side of the monitoring device 10 to further radially restrict the monitoring device 10 in cooperation with the opening 211.

As an optional embodiment, the position limiting surface 222 formed by the interconnection of the second reinforcing portions 2212 may also be in surface contact with the top of the monitoring device 10, so that the second reinforcing portions 2212 may axially restrict the monitoring device 10. It is understood that the accommodating space in this embodiment is designed to be similar to the profile of the monitoring device 10, so that the support structure 220 may sufficiently restrict the monitoring device 10 and prevent the monitoring device 10 from moving relative to the support structure 220, thus better protecting the monitoring device 10.

It is noted that the securing device in this embodiment may be used to restrict and protect the monitoring device 10 of a round shape. When the monitoring device 10 is of other shapes (e.g., square and triangular), the shapes of the accommodating space and the opening 211 are also designed to be adapted to the shape of the monitoring device 10. Continuously referring to FIG. 3, the first reinforcing portion 2211 and the second reinforcing portion 2212 may be integrated in one-piece construction to ensure the strength of the reinforcing rib 221. The reinforcing ribs 221 may also be integrated in one-piece construction, and the reinforcing ribs 221 and the connecting structure 210 may also be integrated in one-piece construction so as to prevent a fracture occurring between the reinforcing ribs 221 and the connecting structure 210 during usage by the user, thus ensuring the overall strength of the support part 200.

In addition, as previously mentioned, two neighboring reinforcing ribs 221 are spaced apart. That is, there is a certain spacing between two neighboring reinforcing ribs 221, so that the support structure 220 is a non-closed structure. In this way, the breathability of the securing device is improved and allergies of the user are reduced.

Also, the first reinforcing portion 2211 may be configured with a hollow 223 in a mid-portion thereof. The hollow 223 may not only enhance a pleasing appearance of the securing device, but also further improve the breathability of the securing device.

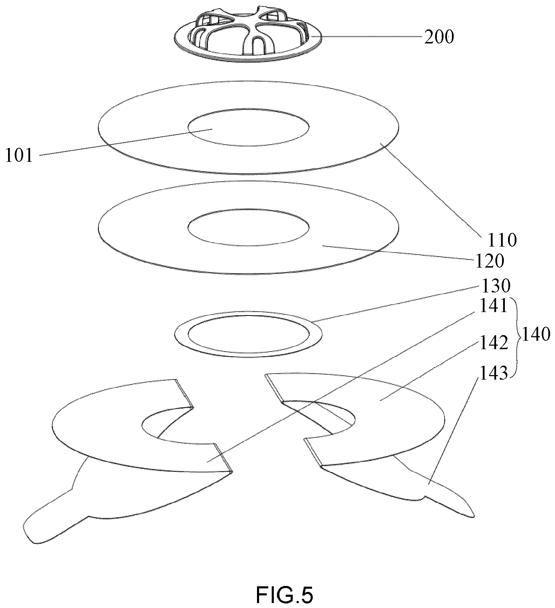

Referring together to FIGs. 4 and 5, the application part 100 may comprise a substrate layer 110, a bonding layer 120, a bonding clearance layer 130 and a release layer 140 stacked in sequence, the above mentioned substrate layer 110, the bonding layer 120, the bonding clearance layer 130 and the release layer 140 are coaxially arranged and respectively configured with an avoidance hole 101. The connecting structure 210 is fixedly connected to a side of the substrate layer 110 which faces away from the bonding layer 120. Exemplarily, a side of the connecting structure 210 that faces towards the substrate layer 110 has a flat surface such that the connecting structure 210 may fit onto the surface of the substrate layer 110. In addition, the connecting structure 210 may also be fixed onto the substrate layer 110 in a welding way, thus increasing a contact area between the connecting structure 210 and the substrate layer 110 and improving a fixing effect therebetween.

The release layer 140 covers the bonding layer 120. When the securing device is not in use, the release layer 140 may ensure the adhesion of the bonding layer 120. Before using a securing device, the release layer 140 may be peeled off from the bonding layer 120, and then the bonding layer 120 can be affixed to the skin surface of the user.

The bonding clearance layer 130 is disposed on a side of the bonding layer 120 which faces away from the substrate layer 110. The bonding layer 120 and the bonding clearance layer 130 are of a ring structure due to the avoidance holes 101. Along the radial direction of the bonding layer 120, an outer ring edge of the bonding clearance layer 130 may be located between an inner ring edge of the bonding layer 120 and an outer ring edge of the bonding layer 120. That is, the bonding clearance layer 130 is only partially affixed to the surface of the bonding layer 120.

In this embodiment, the bonding clearance layer 130 is not adhesive. When the securing device is affixed to the skin surface of the user by means of the bonding layer 120, a portion of the bonding layer 120 that is located at the outer edge of the bonding clearance layer 130 is affixed to the skin surface of the user, and the portion of the bonding layer 120 that is directly opposite to the bonding clearance layer 130 is not adhesive. When the user wears the monitoring device 10, since the monitoring device 10 is also affixed to the skin surface of the user by means of a glue layer, the direct contact between the bonding layer 120 and the glue layer of the monitoring device 10 is avoided by providing the bonding clearance layer 130, at which point the glue layer of the monitoring device 10 and the bonding layer 120 do not overlap on the skin surface of the user. When the securing device is removed from the user body, the bonding layer 120 does not damage the glue layer of the monitoring device 10, thereby avoiding removing the monitoring device 10 along with the securing device when the securing device is removed.

As an optional embodiment, as shown in FIG. 5, the release layer 140 may comprise a first release part 141 and a second release part 142, the first release part 141 and the second release part 142 are symmetrically arranged, the first release part 141 may be affixed to one half of the bonding layer 120, and the second release part 142 may be affixed to the other half of the bonding layer 120. Additionally, the first release part 141 and the second release part 142 may also be provided with a peel-off tab 143, respectively, so that when the release layer 140 is affixed to the bonding layer 120 and the application part 100 fits onto the skin surface of the user, the peel-off tab 143 with an end portion protruding from the outer ring edge of the release layer 140 facilitates the user to manually lift up the peel-off tab 143, thus peeling the first release part 141 or the second release part 142 from the bonding layer 120.

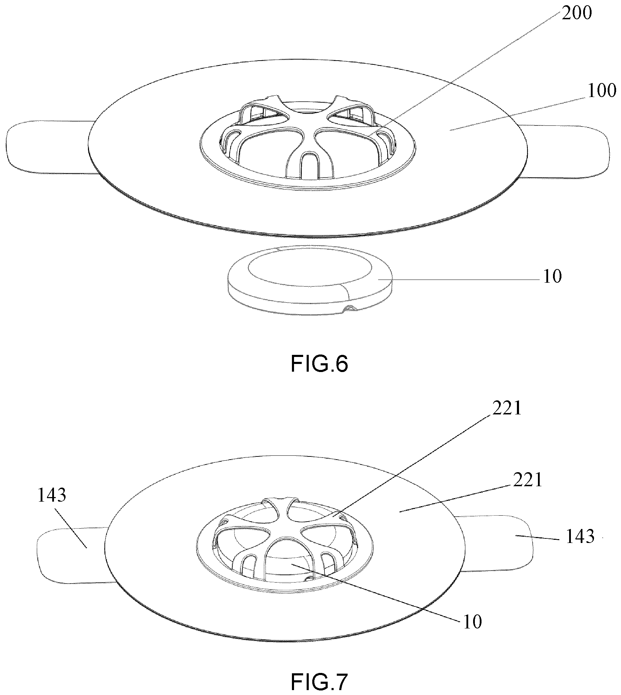

Referring to FIGs. 6 and 7, when using the securing device, the securing device may be firstly used to cover the monitoring device 10 such that the monitoring device 10 is located inside the support structure 220. After that, the first release part 141 or the second release part 142 is peeled off through the peel-off tab 143 to affix an exposed portion of the bonding layer 120 to the skin surface, and then the other release part is peeled off to affix the other exposed portion to the skin surface. In this way, when the securing device is affixed to the skin surface of the user, the alignment of the securing device is facilitated so that the securing device is capable of being affixed to a preset position.

In some embodiments, the material of the substrate layer 110 may be non-woven fabric, etc. The non-woven fabric is soft and achieves a complete fit with the skin surface of the user, thereby improving the adhesive effect of the securing device. The hardness of the support part 200 is greater than that of the application part 100, so that the securing device will not be easily deformed and compress the monitoring device 10 during the usage of the securing device. Exemplarily, the overall material of the support part 200 may be polypropylene, which has a certain degree of flexibility while ensuring a certain degree of strength, so that it is not easily damaged when an external force is applied to the support part 200, thereby ensuring the support strength of the support part 200.

To sum up, the securing device of the present application may improve the protection effect of the monitoring device and guarantee the user experience.

It will be apparent to those skilled in the art that various amendments and variations may be made to the embodiments of the present application without departing from the spirit and scope of the present application. Therefore, the present application is intended to cover these modifications and variations provided that such modifications and variations made to the present application fall within the scope of the claims of the present application and equivalent technologies thereof.

## Claims

1. A securing device, comprising an application part and a support part, wherein the support part is connected to the application part;
wherein the application part is used to affix to the skin surface of the user, and the application part is configured with an avoidance hole for avoiding a monitoring device;
wherein the support part comprises a support structure, an accommodating space is arranged in the interior of the support structure to receive the monitoring device, an opening is configured on a side of the support structure that is close to the application part, and the opening is directly opposite to the avoidance hole; and
wherein the support structure comprises a plurality of reinforcing ribs, the plurality of reinforcing ribs are spaced in a circumferential direction around the first avoidance hole; and along the radial direction of the avoidance hole, a first end of each of the reinforcing ribs is connected to the application part, a second end of each of the reinforcing ribs extends towards a center of the avoidance hole, and the second ends of the reinforcing ribs are interconnected so that the plurality of reinforcing ribs coordinately form the accommodating space.

2. The securing device according to claim 1, wherein at least a portion of each of the reinforcing ribs is in surface contact with the monitoring device when the monitoring device is located in the accommodating space.

3. The securing device according to claim 2, wherein an orthographic projection of the opening on a projection plane coincides with an orthographic projection of the monitoring device on the projection plane, the projection plane is a plane perpendicular to an axis of the avoidance hole.

4. The securing device according to claim 2 or 3, wherein the second ends of the reinforcing ribs are in surface contact with the top of the monitoring device.

5. The securing device according to claim 1, wherein the support part further comprises a connecting structure, the support structure is connected to the application part by means of the connecting structure; and
the connecting structure is configured with the opening, and the first ends of the reinforcing ribs are connected to an inner wall of the opening.

6. The securing device according to claim 5, wherein the connecting structure and the support structure are integrated in one-piece construction.

7. The securing device according to claim 1, wherein the reinforcing ribs are integrated in one-piece construction.

8. The securing device according to claim 1, wherein a portion of each of the reinforcing ribs that is close to the first end is configured with a hollow.

9. The securing device according to claim 1, wherein the application part comprises a substrate layer, a bonding layer, and a release layer stacked in sequence, and the support part is connected to a side of the substrate layer which faces away from the bonding layer, and the release layer covers the bonding layer.

10. The securing device according to claim 9, wherein the application part further comprises a bonding clearance layer, the bonding clearance layer is disposed on a side of the bonding layer which faces away from the substrate layer; and
wherein the bonding layer and the bonding clearance layer are in ring-shaped structures, the bonding layer is coaxially disposed with respect to the bonding clearance layer, and along the radial direction of the bonding layer, an outer ring edge of the bonding clearance layer is located between an outer ring edge of the bonding layer and an inner ring edge of the bonding layer.
